# EUROPEAN PATENT APPLICATION

(11) **EP 4 290 215 A1**
(43) Date of publication of application: **13.12.2023**
(21) Application number: 21923712.0
(22) Date of filing: 03.02.2021
(51) Int. Cl.: G01N 21/00, G01N 33/00

(54) **KIT AND SAMPLE DETECTION DEVICE**

(71) Applicant: Shenzhen Dymind Biotechnology Co., Ltd, Shenzhen, Guangdong 518107 (CN)
(72) Inventor: QIN, Junfang, Shenzhen, Guangdong 518107 (CN); ZHAI, Liuwei, Shenzhen, Guangdong 518107 (CN)
(74) Representative: De Arpe Tejero, Manuel
(86) International application number: PCT/CN2021/075132
(87) International publication number: WO 2022/165684

(57) **Abstract**

A reagent kit is provided and includes a kit body and an assembling seat. The assembling seat includes: an assembling cylinder and a rear-pool electrode. The assembling cylinder defines an axially-extending fluid guiding chamber and a radially-extending fluid outlet communicated with the axially-extending fluid guiding chamber. The radially-extending fluid outlet is configured to discharge a gas or a liquid in the axially-extending fluid guiding chamber. The rear-pool electrode is connected to the assembling cylinder and extending into the axially-extending fluid guiding chamber. The present reagent kit has a novel structure and low cost, and may be used as single-use consumable product. Expensive materials that can be cleaned and used repeatedly may not be required.

## Description

### TECHNICAL FIELD

The present disclosure relates to the field of medical devices, and in particular to a micro-porous slice, a reagent kit, an assembling seat, and a sample detection device.

### BACKGROUND

A blood cell analyzer, also known as an analyzer for cells in blood, a blood cell device, a blood cell counter, and so on, is one of instruments that are highly widely used for clinical detection.

A large portion of components in a traditional blood cell analyzer belong to a cleaning system, and this is because it is important to ensure that a trace of a previously-detected blood sample is cleared before a next blood sample is detected. The entire cleaning system is complex and has a large number of components. In addition, a large volume of reagents is required for a cleaning process, and the cleaning process is time consumed.

Compared to the traditional blood cell analyzer, configuration of components of a point-of-care testing (POCT) blood cell analyzer is greatly simplified. In the POCT blood cell analyzer, components related to a cleaning fluid path in the traditional blood analyzer are completely removed. Therefore, complexity and production cost of the POCT blood cell analyzer is greatly reduced.

However, some functions of the POCT blood cell analyzer in the art are excessively simple, and some structures of the POCT blood cell analyzer in the art are relatively complex. Some POCT blood cell analyzers in the art are less automated, and some POCT blood cell analyzers in the art are expensive.

### SUMMARY OF THE DISCLOSURE

The present disclosure provides a micro-porous slice, a reagent kit, an assembling seat, and a sample detection device, to solve at least part of the above technical problems.

In order to solve the above technical problem, the present disclosure provides a reagent kit, including a kit body and an assembling seat. The assembling seat includes: an assembling cylinder and a rear-pool electrode. The assembling cylinder defines an axially-extending fluid guiding chamber and a radially-extending fluid outlet communicated with the axially-extending fluid guiding chamber. The radially-extending fluid outlet is configured to discharge a gas or a liquid in the axially-extending fluid guiding chamber. The rear-pool electrode is connected to the assembling cylinder and extending into the axially-extending fluid guiding chamber.

The micro-porous slice, the reagent kit, the assembling seat, and the sample detection device in the present disclosure have novel structures and low cost, and may be used as single-use consumable products, expensive materials that can be cleaned and used repeatedly may not be required.

### BRIEF DESCRIPTION OF THE DRAWINGS

In order to more clearly illustrate technical solutions in the embodiments of the present disclosure, the accompanying drawings to be used for describing the embodiments will be briefly introduced in the following. Obviously, the accompanying drawings show only some of the embodiments of the present disclosure, and any ordinary skilled in the art may obtain other drawings based on the following drawings without any creative work.
FIG. 1 is a perspective view of a micro-porous slice according to an embodiment of the present disclosure.
FIG. 2 is a cross-sectional view of the micro-porous slice shown in FIG. 1.
FIG. 3 is an explosive view of a reagent kit, being viewed from a viewing angle, according to an embodiment of the present disclosure.
FIG. 4 is an explosive view of the reagent kit, being viewed from another viewing angle, according to an embodiment of the present disclosure.
FIG. 5 is a cross-sectional view of the reagent kit, being viewed from a viewing angle, according to an embodiment of the present disclosure.
FIG. 6 is a cross-sectional view of the reagent kit, being viewed from another viewing angle, according to an embodiment of the present disclosure.

### DETAILED DESCRIPTION

Technical solutions in the embodiments of the present disclosure will be described clearly and completely in the following by referring to the accompanying drawings in the embodiments of the present disclosure. Obviously, the described embodiments are only a part of, but not all of, the embodiments of the present disclosure. Based on the embodiments in the present disclosure, all other embodiments, which are obtained by any ordinary skilled person in the art without making creative work, shall fall within the scope of the present disclosure.

To be noted that any directional indication in the embodiments of the present disclosure (such as up, down, left, right, front, rear, and so on) is only used to explain a relative positional relationship and a relative movement between various components disposed in a particular attitude (the attitude as shown in the accompanying drawings). The directional indication may be correspondingly changed as the particular attitude changes.

In addition, terms "first", "second" and the like are used in the embodiments of the present disclosure for descriptive purposes only and shall not be interpreted as indicating or implying relative importance or implicitly specifying the number of the indicated technical features. Therefore, any feature defined by the "first" and the "second" may expressly or implicitly include at least one such feature. In addition, technical solutions of various embodiments may be combined with each other, as long as the combination is achievable by any ordinary skilled person in the art. When the combination of technical solutions is contradictory or unachievable, the combination shall be interpreted as non-existing and is not included in the scope of the present disclosure.

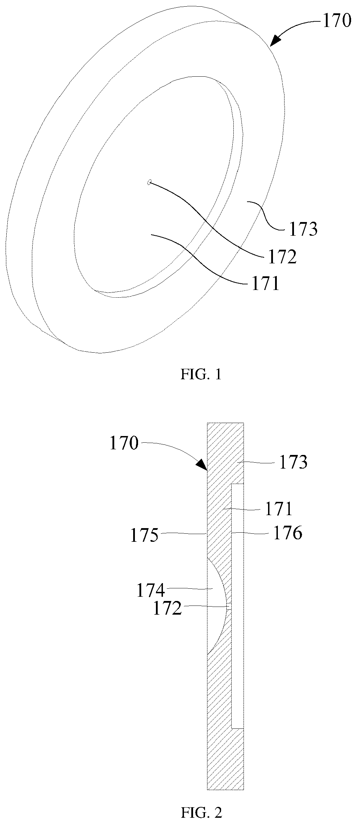

In a first embodiment, as shown in FIG. 1 and FIG. 2, the present disclosure provides a micro-porous slice 170. The micro-porous slice 170 includes a slice body 171. The slice body 171 defines a micro-hole 172 that allows cells to pass through one by one. A size of the micro-hole 172 may be determined based on a particle size of the cell. The slice body 171 is a plastic slice or a ceramic slice. Mechanical strength of the plastic slice or the ceramic slice is relatively weak. In the present embodiment, a reinforcing portion 173 is further arranged on the slice body 171 to ensure the mechanical strength of the micro-porous slice 170 and to facilitate assembling of the micro-porous slice 170. When the micro-porous slice 170 is being assembled, the micro-hole 172 may not be easily contaminated or worn due to contact. The plastic or ceramic material is relatively low cost, and therefore, the micro-porous slice 170 made of the plastic or ceramic may serve as a single-use product, and expensive materials that can be repeatedly cleaned and used may not be required.

The slice body 171 has a first surface 175 and a second surface 176 opposite to the first surface 175. The reinforcing portion 173 is arranged on the first surface 175 and/or the second surface 176.

The reinforcing portion 173 is disposed near an edge of the slice body 171. The reinforcing portion 173 may be a convex ring or the like. The convex ring may be configured as a continuous one-piece structure. Alternatively, a plurality of protrusions cooperatively form the convex ring to surround the edge. An outer edge of the convex ring coincides with or does not coincide with an outer edge of the slice body 171.

The convex ring is connected to the first surface 175 and/or the second surface 176 of the slice body 171 through a vertical surface, an inclined surface, or a curved surface. The inclined surface and the curved surface may reduce a residue of a sample, and detection accuracy is improved.

The first surface 175 of the slice body 171 is arranged with a recessed guiding portion 174 around a periphery of the micro-hole 172. The recessed guiding portion 174 may be spherical or conical.

A ratio of a thickness of the convex ring to a thickness of the slice body 171 is 0.2-2. In some embodiments, a ratio of a width of the convex ring to a radius of the slice body 171 is less than or equal to 1. When the thickness of the convex ring is excessively small, the mechanical strength of the micro-porous slice 170 may not be improved effectively. When the thickness of the convex ring is excessively large, it may be a waste of material. When the thickness of the convex ring is excessively large, a thickness of a wall of the micro-hole 172 of the micro-porous slice 170 may not be easily controlled, and therefore, a manufacturing process may be complicated. The ratio of the width of the convex ring to the radius of the slice body 171 is 0.2-0.8. Similarly, when the width of the convex ring is excessively small, the mechanical strength of the micro-porous slice 170 may not be improved effectively. When the width of the convex ring is excessively large, the thickness of the wall of the micro-hole 172 of the micro-porous slice 170 may not be easily controlled, and therefore, a manufacturing process may be complicated. When the width of the convex ring is excessively large (such as, almost equal to the radius of the slice body 171), an axial channel of the micro-hole 172 may be longer. In this case, particles of a to-be-detected sample may flow reversely, and the detection accuracy, while the cells are passing through the hole, may be affected.

The micro-porous slice 170 in the present embodiment is arranged with the reinforcing portion 173. Therefore, the mechanical strength of the micro-porous slice 170 is improved, and the micro-porous slice 170 made of the plastic or the ceramic may be prevented from being easily deformed when being assembled. In the present embodiment, a reagent kit including the above-mentioned micro-porous slice 170 is provided. Detailed structures of the reagent kit are described in the following.

In a second embodiment, as shown in FIG. 1 to FIG. 6, the present embodiment provides a reagent kit, including a kit body 100, an assembling seat 160, and the micro-porous slice 170.

The kit body 100 includes a front pool 120. The kit body 100 is provided with a front-pool electrode 121 corresponding to the front pool 120. Two front pools 120 may be defined. One of the two front pools 120 may be used for white blood cell (WBC) detection, and the other one of the two front pools 120 may be used for red blood cell (RBC) detection.

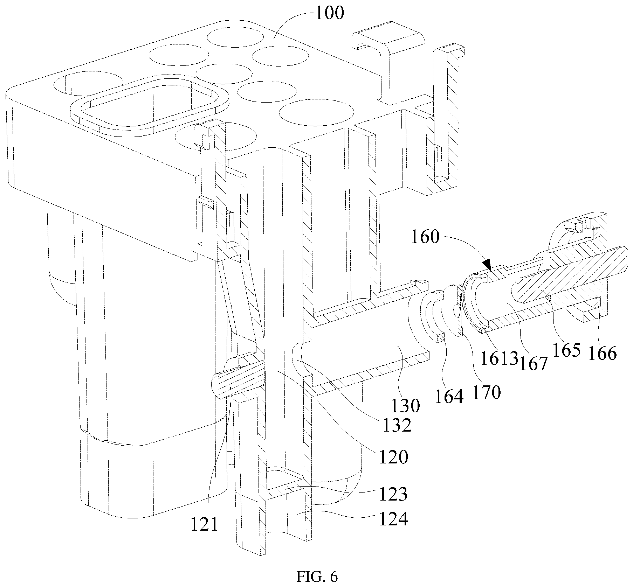

As shown in FIG. 6, the assembling seat 160 is connected to the kit body 100. The assembling seat 160 defines a fluid guiding chamber 167 extending in an axial direction (the fluid guiding chamber 167 extending in the axial direction may be referred to as a rear pool) and includes a rear-pool electrode 165 extending towards the axially-extending fluid guiding chamber 167.

As shown in FIG. 1, FIG. 2, and FIG. 6, the micro-porous slice 170 defines the micro-hole 172 that allows the cells to pass through one by one. The micro-porous slice 170 is disposed between the front pool 120 and the axially-extending fluid guiding chamber 167. The front pool 120 and the axially-extending fluid guiding chamber 167 are communicated with each other through the micro-hole 172. The front-pool electrode 121 and the rear-pool electrode 165 are spaced apart from each other and are respectively disposed at two sides of the micro-porous slice 170.

The assembling seat 160 is removably connected to the kit body 100.

In an embodiment, the front-pool electrode 121 and the kit body 100 may be configured as a one-piece and integral structure, alternatively, the front-pool electrode 121 may be detachably connected to the kit body 100. The rear-pool electrode 165 and the assembling seat 160 may be configured as a one-piece and integral structure, alternatively, the rear-pool electrode 165 may be detachably connected to the assembling seat 160. The front-pool electrode 121 and/or the rear-pool electrode 165 may be a column electrode. A length of the rear-pool electrode 165 is greater than or equal to a length of the front-pool electrode 121. The micro-porous slice 170 and the kit body 100 may be configured as a one-piece and integral structure, alternatively, the micro-porous slice 170 may be detachably connected to the kit body 100. Alternatively, the micro-porous slice 170 and the assembling seat 160 may be configured as a one-piece and integral structure, alternatively, the micro-porous slice 170 may be detachably connected to the assembling seat 160.

The kit body 100 defines a mounting cavity 130. The assembling seat 160 may be snap-fitted, threadly-fitted, interference-fitted, laser-welded, or adhesively-fitted with the mounting cavity 130.

The kit body 100 and/or the assembling seat 160 are plastic. The front-pool electrode 121 is embedded into the kit body 100. An end surface of the front-pool electrode 121 may be aligned with, protruding from, or recessed from an outer surface of the kit body 100. The rear-pool electrode 165 is embedded into the assembling seat 160. An end surface of the rear-pool electrode 165 may be aligned with, protruding from, or recessed from an outer end surface of the assembling seat 160. An outer end of the front-pool electrode 121 and an outer end of the rear-pool electrode 165 (i.e., an end of the front-pool electrode 121 away from the rear-pool electrode 165, and an end of the rear-pool electrode 165 away from the front-pool electrode 121) are configured to be connected to an operating voltage. An inner end of the front-pool electrode 121 and an inner end of the rear-pool electrode 165 (i.e., an end of the front-pool electrode 121 near the rear-pool electrode 165, and an end of the rear-pool electrode 165 near the front-pool electrode 121) contact to-be-detected sample fluid. While performing detection, the axially-extending fluid guiding chamber 167 may be fully filled with the to-be-detected sample fluid.

The reagent kit further includes an inner seal 164. The inner seal 164 is disposed between the micro-porous slice 170 and the front pool 120. Specifically, the inner seal 164 may be disposed between the first surface 175 of the micro-porous slice 170 and the front pool 120, allowing the to-be-detected sample fluid in the front pool 120 to enter the axially-extending fluid guiding chamber 167 through the micro-hole 172 only.

The reagent kit further includes an outer seal 166. The outer seal 166 is disposed between the assembling seat 160 and a free end of the mounting cavity 130.

In an embodiment, the inner seal 164, the outer seal 166, the micro-porous slice 170, and the assembling seat 160 are structures that can be separated from each other.

In another embodiment, the inner seal 164, the outer seal 166, the micro-porous slice 170 and the assembling seat 160 may be configured as a one-piece and integral structure. In this way, the number of assembling elements may be reduced, difficulty of assembling may be reduced, and time consumed for assembling may be reduced. The inner seal 164 and the outer seal 166 may be produced by a secondary injection moulding process, and relatively soft plastic material may be used in the secondary injection moulding process to form the seals.

In another embodiment, the inner seal 164, the outer seal 166, and the assembling seat 160 are configured as a one-piece and integral structure. The micro-porous slice 170 is detachably connected to the one-piece and integral structure. In this way, the number of assembling elements may be reduced, and manufacturing precision and a yield rate of the micro-porous slice 170 may be ensured. The inner seal 164 may be flexible to some extent. When the micro-porous slice 170 is assembled with the one-piece and integral structure, the micro-porous slice 170 may be assembled to a rear of the inner seal 164 due to the flexibility, and the inner seal 164 may still seal the first surface 175 of the micro-porous slice 170.

The reagent kit in the present embodiment has a novel structure and may be easily assembled.

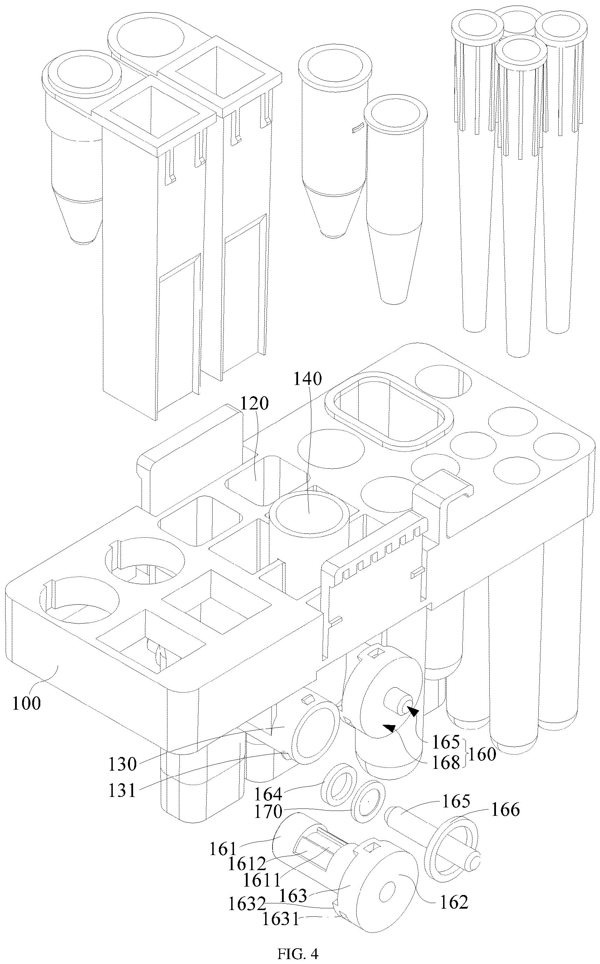

In a third embodiment, as shown in FIG. 1 to FIG. 6, the present embodiment provides an assembling seat 160. The assembling seat 160 includes an assembling cylinder 168 and a rear-pool electrode 165. The assembling cylinder 168 defines the axially-extending fluid guiding chamber 167 and a radially-extending fluid outlet 1611 (as shown in FIG. 4) communicated with the axially-extending fluid guiding chamber 167. The radially-extending fluid outlet 1611 is configured to discharge a gas or a liquid in the axially-extending fluid guiding chamber 167. The rear-pool electrode 165 is connected to the assembling cylinder 168 and extends towards the axially-extending fluid guiding chamber 167.

A first predetermined distance is defined between an inner end of the radially-extending fluid outlet 1611 and an inner end of the assembling cylinder 168. A second predetermined distance is defined between an inner end of the rear-pool electrode 165 and the inner end of the assembling cylinder 168. The first predetermined distance is less than or equal to the second predetermined distance. When the first predetermined distance is less than the second predetermined distance, air bubbles in the axially-extending fluid guiding chamber 167 may be more easily discharged out of the axially-extending fluid guiding chamber 167. When air bubbles are present in the axially-extending fluid guiding chamber 167, the detection accuracy will be affected. The inner end is determined with reference to an interior of the kit body 100. When the front-pool 120 is taken as a reference, an end pointing towards the front pool is determined as the inner end, and an end pointing away from the front pool 120 is determined as an outer end. For example, the inner end of the mounting cavity 130 is communicated with the front pool 120 through the inner end 132. The outer end of the mounting cavity 130 is an opening end, and the assembling seat 160 is received into the mounting cavity 130 from the outer end.

A length of the radially-extending fluid outlet 1611 in the axial direction of the axially-extending fluid guiding chamber 167 is greater than or equal to a length of the rear-pool electrode 165 being received in the axially-extending fluid guiding chamber 167. The inner end of the rear-pool electrode 165 may be or may not be received into the axially-extending fluid guiding chamber 167. That is, the inner end of the rear-pool electrode 165 may protrude from, be aligned with, or be recessed from a bottom surface of the axially-extending fluid guiding chamber 167.

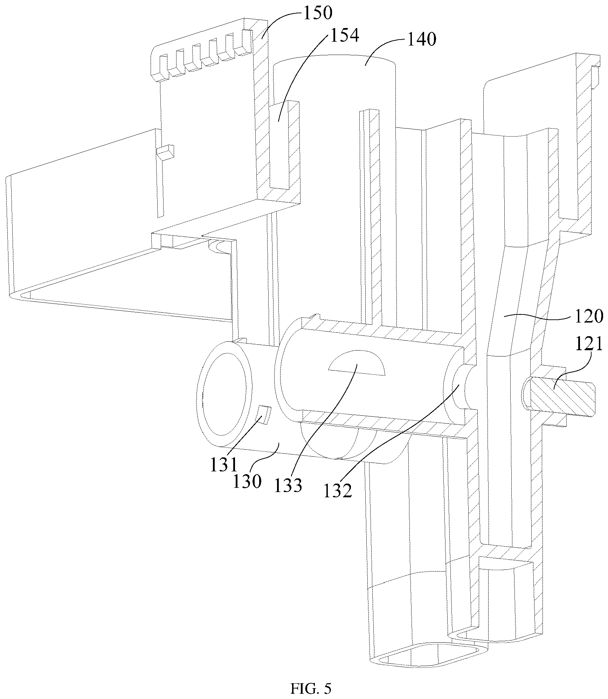

Further, an outer surface of the assembling cylinder 168 defines a recess communicated with the radially-extending fluid outlet 1611, and the recess serves as a fluid guiding slot 1612. The fluid guiding slot 1612 allows the gas or the liquid in the axially-extending fluid guiding chamber 167 to flow through the radially-extending fluid outlet 1611, which is located directly above the axially-extending fluid guiding chamber 167, and to be further discharged by flowing through the fluid guiding slot 1612, which is located obliquely above the axially-extending fluid guiding chamber 167. In this way, two assembling seats 160 may share one pressure pool 140, which is communicated with the two assembling seats 160. The pressure pool 140 is communicated to the mounting cavity 130 through a through hole 133 (such as the fan-shaped structure as shown in FIG. 5). The pressure pool 140 is further communicated to the axially-extending fluid guiding chamber 167 through the fluid guiding slot 1612 and the radially-extending fluid outlet 1611 successively.

The micro-porous slice 170, which allows cells to pass through one by one, is arranged at the inner end of the assembling cylinder 168. Specific structures of the micro-porous slice 170 may be referred to the aforementioned embodiment.

A third predetermined distance is defined between the inner end of the rear-pool electrode 165 and the micro-porous slice 170. The third predetermined distance is 0.2-2 times of the axial length of the axially-extending fluid guiding chamber 167. It has been experimentally verified that, within this range, a better signal accuracy may be achieved while performing impedance-based detection.

The inner end of the assembling cylinder 168 is arranged with a sink 1613. The micro-porous slice 170 is mated with the sink 1613. The reinforcing portion 173 of the micro-porous slice 170 abuts against the sink 1613.

As shown in FIG. 4, the assembling cylinder 168 includes a rear-pool cylinder 161, an end plate 162, and an outer cylinder 163. The rear-pool cylinder 161, the end plate 162, and the outer cylinder 163 are configured as a one-piece and integral structure. The rear-pool cylinder 161 defines the axially-extending fluid guiding chamber 167 and the radially-extending fluid outlet 1611. The end plate 162 radially connects the rear-pool cylinder 161 with the outer cylinder 163. The outer cylinder 163 sleeves an outer periphery of the rear-pool cylinder 161 and is spaced apart from the outer periphery of the rear-pool cylinder 161.

The radially-extending fluid outlet 1611 may be in a circular hole, an elongated round hole, or a rectangular hole.

The present disclosure further provides a reagent kit including a kit body 100 and the assembling seat 160 as described in the above. The kit body 100 defines the front pool 120 and the mounting cavity 130 communicated with the front pool 120. The assembling cylinder 168 is fluidly connected to the mounting cavity 130. The front pool 120 communicated to the axially-extending fluid guiding chamber 167 through the micro-hole 172 of the micro-porous slice 170.

For the reagent kit and the assembling seat 160 in the present embodiment, the distance between the inner end of the radially-extending fluid outlet 1611 and the assembling cylinder 168 and the distance between the inner end of the rear-pool electrode 165 and the inner end of the assembling cylinder 168 are limited, enabling air bubbles in the assembling cylinder 168 to be discharged, preventing the detection accuracy of the impedance-based detection from being affected due to the air bubbles being remained in the assembling cylinder 168.

In a fourth embodiment, as shown in FIG. 1 to FIG. 6, the present embodiment provides an assembling seat 160, including the assembling cylinder 168 and the rear-pool electrode 165. The rear-pool electrode 165 is a column electrode and is connected to the assembling cylinder 168.

The assembling cylinder 168 defines the radially-extending fluid outlet 1611 and is arranged with at least one snap portion 1631. The snap portion 1631 is aligned with the radially-extending fluid outlet 1611. The snap portion 1631 may be a snap hole or a snap bump. Accordingly, an outer surface of the mounting cavity 130 is arranged with a snap block 131 corresponding to the snap hole. An opening direction of one of the at least one snap portion 1631 is consistent with and is parallel to an opening direction of the radially-extending fluid outlet 1611. In this way, while performing the injection moulding process, moulds may be pulled out from a same direction.

The assembling cylinder 168 defines the axially-extending fluid guiding chamber 167. The axially-extending fluid guiding chamber 167 is communicated to the radially-extending fluid outlet 1611. The rear-pool electrode 165 extends into the axially-extending fluid guiding chamber 167.

The rear-pool electrode 165 may be embedded in the assembling seat 160 by the injection moulding process. The end of the rear-pool electrode 165 may be aligned with protruding from or recessed from an outer end surface of the assembling seat 160.

In the preset embodiment, the assembling cylinder 168 includes the rear-pool cylinder 161, the end plate 162, and the outer cylinder 163. The rear-pool cylinder 161, the end plate 162, and the outer cylinder 163 are configured as a one-piece and integral structure. The rear-pool cylinder 161 defines the radially-extending fluid outlet 1611 and the axially-extending fluid guiding chamber 167. The end plate 162 radially connects the rear-pool cylinder 161 with the outer cylinder 163.

A plurality of positioning bumps 1632 may extend from the outer cylinder 163 in the axial direction of the outer cylinder 163 and are spaced apart from each other. The at least one snap portion 1631 and the plurality of positioning bumps 1632 are disposed in one-to-one correspondence with each other.

The first predetermined distance is defined between the inner end of the radially-extending fluid outlet 1611 and the inner end of the assembling cylinder 168, and the second predetermined distance is defined between the inner end of the rear-pool electrode 165 and the inner end of the assembling cylinder 168. The first predetermined distance is less than or equal to the second predetermined distance.

The rear-pool electrode 165 and the assembling cylinder 168 may be configured as the one-piece and integral structure or may be detachably connected to each other.

The inner end of the rear-pool cylinder 161 is arranged with the sink 1613 to be mated with the micro-porous slice 170 that allows cells to pass through one by one.

The present disclosure further provides a reagent kit including the kit body 100 and the assembling seat 160 as described in the above. The kit body 100 defines the front pool 120 and the mounting cavity 130 communicated with the front pool 120. The assembling cylinder 168 of the assembling seat 160 is communicated with the mounting cavity 130. The front pool 120 is communicated with the axially-extending fluid guiding chamber 167 through the micro hole 172 in the micro-porous slice 170.

For the reagent kit and the assembling seat 160 in the present embodiment, the radially-extending fluid outlet 1611 is aligned with the snap portion 1631, moulds may be easily pulled out after performing the injection moulding process.

In a fifth embodiment, as shown in FIG. 1 to FIG. 6, the present embodiment provides an assembling seat 160, including the assembling cylinder 168 and the rear-pool electrode 165.

The assembling cylinder 168 defines the axially-extending fluid guiding chamber 167 and the radially-extending fluid outlet 1611 that are communicated with the axially-extending fluid guiding chamber 167. The outer surface of the assembling cylinder 168 defines the recess that is communicated to the radially-extending fluid outlet 1611 to serve as the fluid guiding slot 1612. The fluid guiding slot 1612 allows the gas or the liquid in the axially-extending fluid guiding chamber 167 to flow through the radially-extending fluid outlet 1611 (which for example may be located directly above the axially-extending fluid guiding chamber 167) and to further flow through the fluid guiding slot 1612 (which for example may be located obliquely above the axially-extending fluid guiding chamber 167) to smoothly flow to reach the pressure pool 140. In this way, two assembling seats 160 may share one pressure pool 140, which is communicated with the two assembling seats 160. The pressure pool 140 is communicated to the mounting cavity 130 through a through hole 133 (such as the fan-shaped structure as shown in FIG. 5). The pressure pool 140 is further communicated to the axially-extending fluid guiding chamber 167 through the fluid guiding slot 1612 and the radially-extending fluid outlet 1611 successively.

The rear-pool electrode 165 is connected to the assembling cylinder 168 and extends into the axially-extending fluid guiding chamber 167.

The fluid guiding slot 1612 is circumferentially disposed along the outer surface of the assembling cylinder 168. The fluid guiding slot 1612 is curvedly extending. A central angle of the curvedly-extending fluid guiding slot is 0°-360°. In this way, the sample liquid may flow to the pressure pool 140 more smoothly. A depth of the fluid guiding slot 1612 being recessed away from the outer surface of the assembling cylinder 168 is 1/5~4/5 of a thickness of a wall of the assembling cylinder 168. In this way, the liquid may be discharged smoothly, and at the same time, a wall of the fluid guiding slot 1612 has certain mechanical strength and may not be easily damaged. The radially-extending fluid outlet 1611 is fluidly connected to the fluid guiding slot 1612 by a transition surface. The transition surface is a vertical surface, an inclined surface, or a curved surface.

The rear-pool electrode 165 is embedded in the assembling seat 160 and is aligned with, protruding from or recessed from the outer end surface of the assembling seat 160.

The first predetermined distance is between the inner end of the radially-extending fluid outlet 1611 and the inner end of the assembling cylinder 168. The second predetermined distance is between the inner end of the rear-pool electrode 165 and the inner end of the assembling cylinder 168. The first predetermined distance is less than or equal to the second predetermined distance.

As shown in FIG. 4, the assembling cylinder 168 includes the rear-pool cylinder 161, the end plate 162, and the outer cylinder 163. The rear-pool cylinder 161, the end plate 162, and the outer cylinder 163 are configured as a one-piece and integral structure. The rear-pool cylinder 161 defines the radially-extending fluid outlet 1611 and the axially-extending fluid guiding chamber 167. The end plate 162 radially connects the rear-pool cylinder 161 with the outer cylinder 163.

A plurality of positioning bumps 1632 may extend from the outer cylinder 163 in the axial direction of the outer cylinder 163 and are spaced apart from each other. The at least one snap portion 1631 and the plurality of positioning bumps 1632 are disposed in one-to-one correspondence with each other.

The present disclosure further provides a reagent kit includes the kit body100 and the assembling seat 160 as described in the above. The kit body 100 defines the front pool 120 and the mounting cavity 130 communicated with the front pool 120. The assembling cylinder 168 of the assembling seat 160 is communicated with the mounting cavity 130. The front pool 120 is communicated with the axially-extending fluid guiding chamber 167 through the micro hole 172 in the micro-porous slice 170.

For the reagent kit and the assembling seat 160 in the present embodiment, the assembling cylinder 168 defines the radially-extending fluid outlet 1611, and an outer surface of the radially-extending fluid outlet 1611 defines a recess to serve as the fluid guiding slot 1612. In this way, two assembling seats 160 may share one pressure pool 140, which is communicated with the two assembling seats 160, and the pressure pool 140 may guide a flowing direction of the fluid based on the negative pressure.

In a sixth embodiment, as shown in FIG. 1 to FIG. 6, the present embodiment provides an assembling seat 160 that includes the assembling cylinder 168 and the rear-pool electrode 165.

The assembling cylinder 168 is a plastic assembling cylinder and defines the axially-extending fluid guiding chamber 167 and the radially-extending fluid outlet 1611 communicated with the axially-extending fluid guiding chamber 167. The radially-extending fluid outlet 1611 is configured to discharge the gas or the liquid in the axially-extending fluid guiding chamber 167. The rear-pool electrode 165 is connected to the assembling cylinder 168 and extends into the axially-extending fluid guiding chamber 167.

Further, the outer surface of the assembling cylinder 168 defines the recess communicated with the radially-extending fluid outlet 1611, and the recess serves as a fluid guiding slot 1612. The fluid guiding slot 1612 allows the gas or the liquid in the axially-extending fluid guiding chamber 167 to flow through the radially-extending fluid outlet 1611 (which for example may be located directly above the axially-extending fluid guiding chamber 167) and to further flow through the fluid guiding slot 1612 (which for example may be located obliquely above the axially-extending fluid guiding chamber 167) to smoothly flow to reach the pressure pool 140. In this way, two assembling seats 160 may share one pressure pool 140, which is communicated with the two assembling seats 160. The pressure pool 140 is communicated to the mounting cavity 130 through a through hole 133 (such as the fan-shaped structure as shown in FIG. 5). The pressure pool 140 is further communicated to the axially-extending fluid guiding chamber 167 through the fluid guiding slot 1612 and the radially-extending fluid outlet 1611 successively.

The assembling cylinder 168 is arranged with at least one snap portion 1631. The snap portion 1631 is aligned with the radially-extending fluid outlet 1611. The snap portion 1631 may be a snap hole. Since the snap portion 1631 is aligned with the radially-extending fluid outlet 1611, moulds may be pulled out from a same direction while performing the injection moulding process.

The first predetermined distance is defined between the inner end of the radially-extending fluid outlet 1611 and the inner end of the assembling cylinder 168. The second predetermined distance is defined between the inner end of the rear-pool electrode 165 and the inner end of the assembling cylinder 168. The first predetermined distance is less than or equal to the second predetermined distance. When the first predetermined distance is less than the second predetermined distance, air bubbles in the axially-extending fluid guiding chamber 167 may be more easily discharged out of the axially-extending fluid guiding chamber 167. When air bubbles are present in the axially-extending fluid guiding chamber 167, the detection accuracy will be affected.

A length of the radially-extending fluid outlet 1611 in the axial direction of the axially-extending fluid guiding chamber 167 is greater than or equal to a length of the rear-pool electrode 165 being received in the axially-extending fluid guiding chamber 167.

The micro-porous slice 170, which allows cells to pass through one by one, is arranged at the inner end of the assembling cylinder 168.

The third predetermined distance is defined between the inner end of the rear-pool electrode 165 and the micro-porous slice 170. The third predetermined distance is 0.2-2 times of the axial length of the axially-extending fluid guiding chamber 167. The inner end of the assembling cylinder 168 is arranged with a sink 1613. The micro-porous slice 170 is mated with the sink 1613.

In the present embodiment, the assembling cylinder 168 includes the rear-pool cylinder 161, the end plate 162, and the outer cylinder 163. The rear-pool cylinder 161, the end plate 162, and the outer cylinder 163 are configured as a one-piece and integral structure. The rear-pool cylinder 161 defines the axially-extending fluid guiding chamber 167 and the radially-extending fluid outlet 1611. The end plate 162 radially connects the rear-pool cylinder 161 with the outer cylinder 163. The outer cylinder 163 sleeves an outer periphery of the rear-pool cylinder 161 and is spaced apart from the outer periphery of the rear-pool cylinder 161.

The present disclosure further provides a reagent kit including the kit body 100 and the assembling seat 160 as described in the above. The kit body 100 defines the front pool 120 and the mounting cavity 130 communicated with the front pool 120. The assembling cylinder 168 of the assembling seat 160 is fluidly connected to the mounting cavity 130. The front pool 120 communicated to the axially-extending fluid guiding chamber 167 through the micro-hole 172 of the micro-porous slice 170. The front pool 120, the mounting cavity 130, the axially-extending fluid guiding chamber 167, the radially-extending fluid outlet 1611, and the pressure pool 140 cooperatively serve as a fluid guiding channel. The axially-extending fluid guiding chamber 167 may receive the negative pressure to suck the to-be-detected sample in the front pool 120 through the micro-hole 172 of the micro-porous slice 170.

The present embodiment provides a reagent kit and the assembling seat 160, which has a novel structure and may be easily manufactured and assembled.

In a seventh embodiment, as shown in FIGS. 1 to 6, the present embodiment provides a reagent kit, the reagent kit includes a kit body 100. The kit body 100 defines a hemolytic agent pool 106 and a diluent pool 111 disposed near the hemolytic agent pool 106.

At least two of the hemolytic agent pool 106, the diluent pool 111, and the kit body 100 are configured as a one-piece and integral structure. At least two of the hemolytic agent pool 106, the diluent pool 111, and the kit body 100 are detachably connected with each other. That is, the hemolytic agent pool 106, the diluent pool 111, and the kit body 100 may be a one-piece structure or may be separated from each other.

For example, the kit body 100, the hemolytic agent pool 106, and the diluent pool 111 are three separated structure and may be detachably connected to each other. Alternatively, the diluent pool 111 and the kit body 100 are configured as a one-piece and integral structure, and a hemolytic agent container 206 may be a separated structure and may be detachably connected to the hemolytic agent pool 106. Alternatively, the hemolytic agent pool 106 and the kit body 100 are configured as a one-piece and integral structure, and a diluent test tube may be detachably assembled to the diluent pool 111. Alternatively, the hemolytic agent pool 106 and the diluent pool 111 are configured as a one-piece and integral structure, and one-piece and integral structure may be detachably assembled to the kit body 100.

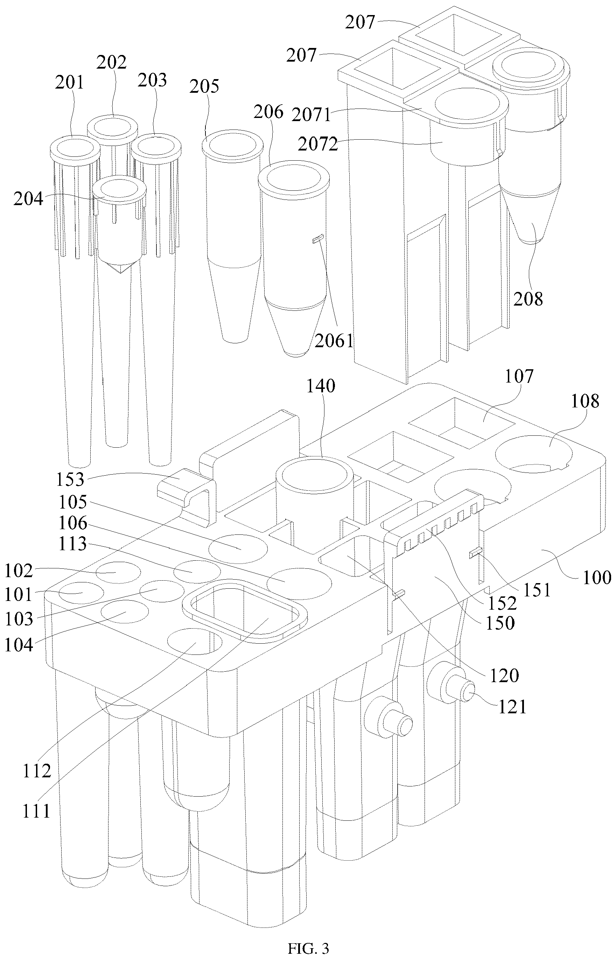

Any two of the hemolytic agent pool 106, the diluent pool 111, and the kit body 100 form a combined component. The combined component is arranged with an assembly portion (for example, the assembly portion may be an inner peripheral wall of a insertion hole defined in the kit body 100). The rest one of the hemolytic agent pool 106, the diluent pool 111, and the kit body 100 is arranged with a disassembly portion (for example, an outer peripheral wall of the hemolytic agent container 206 as shown in FIG. 3). The disassembly portion is engaged with the assembly portion.

A surface of the assembly portion or a surface of the disassembly portion is further arranged with a protrusion 2061. The protrusion may enable the hemolytic agent container 206 to tightly abut against the wall of the insertion hole defined in the kit body 100, such that the hemolytic agent container 206 is tightly engaged with the insertion hole.

In an embodiment, the kit body 100 is arranged with two assembly positions. The hemolytic agent pool 106 and the diluent pool 111 are assembled in the two assembly positions respectively.

In the present embodiment, the kit body 100 defines the front pool 120 and the mounting cavity 130 communicated with the front pool 120. The front pool 140 is communicated to the mounting cavity 130 through a through hole 132 (as shown in FIG. 5 and FIG. 5). The front-pool electrode 121 is assembled in the front pool 120. The reagent kit further includes the assembling seat 160 connected to the mounting cavity 130. The assembling seat 160 is arranged with the micro-porous slice 170 and the front-pool electrode 165. The micro-porous slice 170 is disposed at the through hole 132. The front-pool electrode 121 and the rear-pool electrode 165 are spaced apart from each other and are respectively disposed at two sides of the micro-porous slice 170. The assembling seat 160 includes the assembling cylinder 168. The assembling cylinder defines the axially-extending fluid guiding chamber 167 and the radially-extending fluid outlet 1611.

In the present embodiment, the assembling cylinder 168 includes the rear-pool cylinder 161, the end plate 162, and the outer cylinder 163. The rear-pool cylinder 161, the end plate 162, and the outer cylinder 163 are configured as a one-piece and integral structure. The rear-pool cylinder 161 defines the axially-extending fluid guiding chamber 167 and the radially-extending fluid outlet 1611. The end plate 162 radially connects the rear-pool cylinder 161 with the outer cylinder 163. The outer cylinder 163 sleeves an outer periphery of the rear-pool cylinder 161 and is spaced apart from the outer periphery of the rear-pool cylinder 161.

Since usually a large volume of the diluent may be consumed, a volume of the diluent pool 111 in the present embodiment may be greater than or equal to a volume of the hemolytic agent pool 106.

A sealing film is arranged at each of an opening end of the diluent pool 111 and an opening end of the hemolytic agent pool 106, such that the diluent and the hemolytic agent may be stored for a long period of time.

In the present embodiment, the reagent kit 100 has a novel structure, may be detached in various manner, and may be applicable for various detection demands.

In an eighth embodiment, as shown in FIG. 1 to FIG. 6, the present embodiment provides a reagent kit. The reagent kit includes a kit body 100 and at least one item detection pool. The at least one item detection pool and the kit body 100 may be configured as a one-piece and integral structure.

The item detection pool includes a first item detection pool, configured to perform a first item detection, and a second item detection pool, configured to perform a second item detection. The kit body 100 and at least one of the first item detection pool and the second item detection pool may be configured as a one-piece and integral structure, or may be detachably connected with each other.

The reagent kit further includes a third item detection pool, configured to perform a third item detection. The kit body 100 and the third item detection pool may be configured as a one-piece and integral structure, or may be detachably connected with each other.

The second item detection pool and the third item detection pool are disposed on a same side of the first item detection pool. Alternatively, the second item detection pool and the third item detection pool are respectively disposed at two sides of the first item detection pool.

The item detection performed in the first item detection pool, the second item detection pool, and the third item detection pool may be specific protein detection, biochemical detection, immunoassay detection, and blood routine detection. For example, the blood routine detection may be performed cooperatively through the front pool 120, the mounting cavity 130, the assembling seat 160, and the pressure pool 140. The specific protein detection, the biochemical detection, and the immunoassay detection may be performed cooperatively through the above pools and an optical detection cup.

The item detection performed in the first item detection pool, the item detection performed in the second item detection pool, and the item detection performed in the third item detection pool may be the same as each other, completely different from each other, or partially different from each other. Alternatively, any one of the first item detection pool, the second item detection pool, and the third item detection pool supports more than two item detections.

The kit body 100 defines an insertion hole. The insertion hole may include a rectangular insertion hole 107 and a circular insertion hole 108. The second item detection pool is inserted into the insertion hole, or the second item detection pool is arranged with a flange to be hooked on an edge of the insertion hole.

The second item detection pool includes a first pool body 207, a second pool body 208, and a connector 2071 connecting the first pool body 207 with the second pool body 208. The connector 2071 allows the first pool body 207 and the second pool body 208 to form an assembly, and the entire assembly is connected to the kit body 100.

Each of the first item detection pool and/or the second item detection pool is a transparent plastic pool or a glass pool. A sealing film is arranged at each of the opening end of the first item detection pool and/or the opening end of the second item detection pool. While in use, the sealing film may be pierced by a piercing head 204 arranged on the kit body 100, and the detection pools may be used.

The present embodiment provides a reagent kit, which has a novel structure, may be detached in various manner, and may be applicable for various detection demands.

In a ninth embodiment, as shown in FIG. 1 to FIG. 6, the present embodiment provides a reagent kit. The reagent kit includes a kit body 100. The kit body 100 includes a front pool 120, a mounting cavity 130, and a pressure pool. The front pool 120 and the mounting cavity 130 are communicated with each other. The pressure pool 140 is communicated with the mounting cavity 130. The pressure pool 140 is extending vertically, and the mounting cavity 130 is extending horizontally.

An opening end of the mounting cavity 130 faces towards an outer surface of the kit body 100. The reagent kit further includes the assembling seat 160. The assembling seat 160 is fluidly connected to the mounting cavity 130. The assembling seat 160 defines the axially-extending fluid guiding chamber 167, and the axially-extending fluid guiding chamber 167 is communicated to the pressure pool 140.

A fluid guiding gap is defined between the mounting cavity 130 and the assembling seat 160. The axially-extending fluid guiding chamber 167 is communicated to the pressure pool 140 through the fluid guiding gap.

The assembling seat 160 is defines the radially-extending fluid outlet 1611. The axially-extending fluid guiding chamber 167 is communicated to the pressure pool 140 through the radially-extending fluid outlet 1611. The assembling seat 160 defines the fluid guiding slot 1612 near the radially-extending fluid outlet 1611. The axially-extending fluid guiding chamber 167 is communicated to the pressure pool 140 through the radially-extending fluid outlet 1611 and the fluid guiding slot 1612.

The assembling seat 160 is arranged with the micro-porous slice 170 and the rear-pool electrode 165. The front pool 120 is arranged with the front-pool electrode 121. The front-pool electrode 121 and the rear-pool electrode 165 are spaced apart from each other and are respectively disposed at two sides of the micro-porous slice 170.

The assembling seat 160 may be snap-fitted, threadly-fitted, interference-fitted, laser-welded, or adhesively-fitted with the mounting cavity 130.

The reagent kit further includes the inner seal 164 and the outer seal 166. The inner seal 164 is disposed between the micro-porous slice 170 and the kit body 100. The outer seal 166 is disposed between the assembling seat 160 and the kit body 100.

In the present embodiment, two front pools 120 and two mounting cavities 130 are arranged. One pressure pool 140 is communicated with the two mounting cavities 130.

The present embodiment provides a reagent kit having a novel structure. The front pool 120, the mounting cavity 130, and the pressure pool 140 of the reagent kit are communicated with and are perpendicular to each other. The opening end of the pressure pool 140 is located on an upper surface of the kit body 100. A negative pressure source may be applied from a top of the kit body 100. In this way, a structure of the kit body 100 is simplified, preventing a side of the kit body 100 from having a relatively large number of protruding structures.

In a tenth embodiment, as shown in FIG. 1 to FIG. 6, the present embodiment provides a reagent kit. The reagent kit includes a plurality of first pool positions. Centers of the plurality of first pool positions may substantially form a first straight line or a first arc.

The reagent kit further includes a plurality of second pool positions. Centers of the plurality of second pool positions may substantially form a second straight line. The first straight line and the second straight line are spaced apart from each other. The first straight line may be parallel to or perpendicular to the second straight line.

In some embodiments, the reagent kit further includes a plurality of second pool positions. Centers of the plurality of second pool positions may substantially form a second arc. The first arc and the second arc are spaced apart from and are parallel to each other. Each of the first arc and the second arc may be an arc corresponding to a center angle of 0 to 360 degrees.

The plurality of first pool positions and/or the plurality of second pool positions include an impedance detection pool and/or an optical detection pool.

The plurality of first pool positions and/or the plurality of second pool positions further include the diluent pool 111. The diluent pool 111 is configured to encapsulate the diluent. The plurality of first pool positions and/or the plurality of second pool positions further include a hemolytic agent pool 106. The hemolytic agent pool 106 is configured to encapsulate a hemolytic agent. Alternatively, the plurality of first pool positions and/or the plurality of second pool positions further include a sample dilution pool 112, the sample dilution pool 112 is configured to dilute the sample.

The impedance detection pool has a light-transmittance detection window to enable optical detection. That is, one of the two front pools 120 has the light-transmittance detection window to enable optical detection. The entire front pool 120 may be made of transparent plastic. A light-transmission level and a degree of finish of the light-transmittance detection window may be equal to that of the front pool 120 or may be higher than that of the rest part of the front pool 120.

The plurality of first pool positions and/or the plurality of second pool positions further include a plurality of insertion holes.

The plurality of first pool positions and/or the plurality of second pool positions include at least one pipette tip receiving pool (101, 102, 103) and/or at least one sample receiving pool 105.

The plurality of first pool positions and/or the plurality of second pool positions include at least one piercing head receiving pool 104. The reagent kit in the present embodiment may be a biodegradable plastic reagent kit. Since the plurality of pool positions are arranged in a straight line, a pipetting device may move for a shorter path while an automated detection is being performed. The pipetting device is configured to transfer and mix the fluid in each pool.

In an eleventh embodiment, as shown in FIG. 1 to FIG. 6, the present embodiment provides a reagent kit, the reagent kit includes the kit body 100. The kit body 100 has a detection region (such as a region formed by the front pool 120, the mounting chamber 130, the pressure pool 140, and the assembling seat 160), a reagent region (such as a region formed by the diluent pool 111, the hemolytic agent pool 106, and the sample receiving pool 105), an accessory placement region (such as a region formed by the pipette tip receiving pools 101, 102, 103, and the piercing head receiving pool 104).

The kit body 100 further includes an expandable region (such as a region formed by the rectangular insertion hole 107 and the circular insertion hole 108 in the drawings). The expandable region is located near the detection region. The expandable region is located on a side of the detection region or a side of the reagent region.

The accessory placement region is located near the reagent region. The detection region is located near the reagent region.

The detection region includes the impedance detection pool and/or the optical detection pool. The optical detection pool includes a pool configured to detect any one of HGB, CRP, and SAA parameters.

The reagent region includes at least one of the diluent pool, the hemolytic agent pool, and the sample receiving pool 105.

The reagent region includes a pool region. At least one of the diluent container, the hemolytic agent container 206, and the sample receiving pool 105 may be arranged at the pool region.

The accessory placement region includes positions 201, 202, 203 for placing pipette tips and/or a position 204 for placing a piercing head.

The expandable region includes the optical detection pool and/or the impedance detection pool, which are detachably arranged. The optical detection pool includes a pool configured to detect any one of HGB, CRP, and SAA parameters

The reagent kit provided in the present embodiment is a biodegradable plastic reagent kit. Since the reagent kit is divided into various regions, the pipetting device may move for a shorter path while the automated detection is being performed.

In a twelfth embodiment, as shown in FIG. 1 to FIG. 6, the present embodiment provides a reagent kit including the kit body 100. The kit body 100 has the detection region (such as a region formed by the front pool 120, the mounting chamber 130, the pressure pool 140, and the assembling seat 160), the reagent region (such as a region formed by the diluent pool 111, the hemolytic agent pool 106, and the sample receiving pool 105), the accessory placement region (such as a region formed by the pipette tip receiving pools 101, 102, 103, and the piercing head receiving pool 104).

The kit body 100 further includes an expandable region (such as a region formed by the rectangular insertion hole 107 and the circular insertion hole 108 in the drawings). The expandable region is located near the detection region. Alternatively, the expandable region is located on a side of the detection region or a side of the reagent region.

The reagent region includes a plurality of reagent pools. The plurality of reagent pools are arranged into a substantially straight line or an arc.

The detection region includes the impedance detection pool and/or the optical detection pool. The optical detection pool includes a pool configured to detect any one of HGB, CRP, and SAA parameters.

The reagent region includes at least two pool regions. The at least two pool regions including a pool having a bottom and/or an insertion hole without a bottom.

The accessory region includes one or more bottom-having pools, the bottom-having pools are configured receive the pipette tips 201, 202, 203 and/or the piercing head 204.

The circular insertion hole 108 and the rectangular insertion hole 107 are located in the expandable region. Each of the circular insertion hole 108 and the rectangular insertion hole 107 is configured to receive the detection pool. The detection pool is the optical detection pool and/or the impedance detection pool.

The reagent kit in the present embodiment is a biodegradable plastic reagent kit. Since the reagent kit is divided into various regions, the pipetting device may move for a shorter path while the automated detection is being performed.

In a thirteenth embodiment, as shown in FIG. 1 to FIG. 6, the present embodiment provides a reagent kit including the kit body 100. The reagent kit is configured to detect samples. The kit body 100 is arranged with a support portion to enable the reagent kit to be stably supported on a bearing surface.

In an embodiment, the kit body 100 includes two support plates (not shown in the drawings). The two support plates serve as the support portion. An identifier may be arranged on the support plates. The identifier may be a barcode, a QR code or an identification chip, configured to record relevant parameters of the reagent kit.

The kit body 100 is arranged with at least one support platform or at least one support surface near a gravitational center of the reagent kit. The support platform or support surface serves as the support portion.

In an embodiment, the kit body 100 is arranged with three support protrusions. The three support protrusions cooperatively form a triangle. A projection of the gravitational center of the reagent kit falls within a region enclosed by the triangle.

In an embodiment, the kit body 100 includes at least one pool body, a bottom of the pool body may serve as the support portion.

In some embodiments, the bottom of the pool body and any one of the support plates, the support platform, the support surface, and the support protrusions cooperatively serves as the support portion.

The pool body includes a receiving chamber and an extension 124 extending downwardly from the receiving chamber (as shown in FIG. 6, a structure below the bottom 123 of the front pool 120). The extension 124 serves as the support portion. The receiving chamber may receive reagents, samples, or accessories (201-204).

The kit body 100 in the present embodiment is a biodegradable plastic kit body. The reagent kit is arranged with the impedance detection pool and/or the optical detection pool.

The reagent kit in the present embodiment is arranged with a larger number of pool bodies. Bottoms of the pool bodies and/or additional support portions may cooperatively support the reagent kit stably, and the reagent kit is prevented from falling over.

In a fourteenth embodiment, as shown in FIG. 1 to FIG. 6, the present embodiment provides a reagent kit including the kit body 100. The kit body 100 includes a plurality of pool bodies. Each pool body includes a receiving chamber and a support portion connected to the receiving chamber. The support portion is solid or hollow. The solid or hollow support portion may increase a relative height of the bottom wall of the receiving chamber, enabling the reagents to be sucked up easily. A corresponding pipetting device may not be inserted excessively deeply, such that a volume of pre-stored reagents may be saved. When the support portion is solid, the gravitational center of the kit body 100 may be shifted downwardly, and the kit body 100 may be more stable. When the support portion is hollow, the amount of the plastic for manufacturing the kit body 100 may be reduced.

A height of the support portion is greater than or equal to a thickness of the wall of the receiving chamber. The bottom of the receiving chamber may be conical. Specifically, the bottom of the receiving chamber may be curved conical, triangular conical, or polygonal conical. The conical bottom may be extending into a hollow region at the bottom of the support portion. Alternatively, the bottom of the receiving chamber is flat. The receiving chamber is connected to the support portion via the flat bottom.

When the support portion is hollow, the bottom of the support portion has an opening end or is arranged with an enclosed end.

The wall of the receiving chamber and the support portion may be configured as a one-piece and integral structure or may be detachably connected with each other.

When the support portion is hollow, a cross section of the hollow structure may be square, round, polygonal or irregularly shaped. The height of the support portion is 0.1 to 0.8 times of the height of the pool body.

The above-mentioned pool bodies may specifically be any pool, such as the reagent pool, the detection pool, the sample dilution pool 112, or the accessory placement pool (101-104).

The above-mentioned detection pool is configured to perform impedance detection or optical detection. The present embodiment further provides a sample detection device including the above-mentioned reagent kit and a detection seat engaged with the reagent kit. The detection seat is configured to analyze and detect the samples. The detection seat is arranged with a power supplying assembly electrically connected to the front-pool electrode 121 and the rear-pool electrode 165. The detection seat is further arranged with optical detection assemblies disposed on opposing sides of the optical detection pool (such as the first pool body 207).

In a fifteenth embodiment, as shown in FIG. 1 to FIG. 6, the present embodiment provides a reagent kit including the kit body 100. The kit body 100 has the accessory placement regions (101-104), and accessories (201-204) are placed in the accessory placement regions.

A leakage prevention structure is arranged below the accessory placement region. The leakage prevention structure may be a bottom-having pool. The leakage prevention structure and the kit body 100 may be configured as a one-piece and integral structure or may be detachably connected to each other. The accessory placement regions have placement holes, and the accessories (201-204) are placed in the placement holes respectively.

For each placement hole and the respective one of the accessories (201-204) received in the placement hole, a diameter of the placement hole is smaller than or equal to a maximum radial size of the accessory, preventing the accessory (201-204) from being completely received into the placement hole, and preventing the bottom of the accessory (201-204) from being deformed due to contacting the bottom of the pool body when the accessory (201-204) is inserted into the placement hole.

The kit body 100 defines accommodating cavities corresponding to the placement holes. The accessories (201-204) are received in the accommodating cavities respectively. Bottoms of the accommodating cavities are sealed.

Walls of the accommodating cavities and the kit body 100 may be configured as a one-piece and integral structure, or may be detachably connected with each other.

For each accommodating cavity and the respective one of the accessories (201-204) received in the accommodating cavity, a depth of an inner cavity of the accommodating cavity is greater than or equal to a length of the accessory (201-204) being received into the accommodating cavity. In this way, the accessory (201-204), when being placed in the accommodating cavity, is prevented from protruding excessively far away from a surface of the kit body 100. Further, the bottom of the accessory (201-204) is prevented from being deformed due to contacting the bottom of the pool body when the accessory (201-204) is inserted into the placement hole.

For each accommodating cavity and the respective one of the accessories (201-204) received in the accommodating cavity, the length of the accessory (201-204) extending into the accommodating cavity is 0.4 to 1 times of the length of the accommodating cavity.

The present embodiment further provides a sample detection device including the above-mentioned reagent kit and the detection seat engaged with the reagent kit. The detection seat is configured to analyze and detect samples.

For the reagent kit in the present embodiment, when the accessories are discarded, since the bottom-having pool is configured to receive the accessories, residual fluid carried on any surface of the accessories may be prevented from being leaked.

In a sixteenth embodiment, as shown in FIG. 1 to FIG. 6, the present embodiment provides a detection cup assembly. The detection cup assembly includes a first pool body 207 and a second pool body 208 connected to the first pool body 207. The first pool body 207 and the second pool body 208 are connected with each other via a connector 2071. The connector 2071 and at least one of the first pool body 207 and the second pool body 208 are configured as a one-piece and integral structure, or are detachably connected with each other.

The connector 2071 is arranged with at least one cup holder 2072. The cup holder 2072 is detachably connected to the first pool body 207 or the second pool body 208.

In an embodiment, the connector 2071 is arranged with one cup holder 2072, and the connector 2071 and the first pool body 207 are configured as a one-piece and integral structure. The cup holder 2072 defines a placement hole. A shape of placement hole is the same as or different from a shape of a cross section of the first pool body 207.

In an embodiment, the connector 2071 is arranged with two cup holders 2072. One of the two cup holders 2072 has a first placement hole, and the other one of the two cup holders 2072 has a second placement hole. A shape of the first placement hole is the same as or different from a shape of the second placement hole.

The first pool body 207 is a detection cup, and the second pool body 208 is a reagent cup. While performing detection, the reagent in the second pool body 208 is added to the first pool body 207, and detection may be performed after the to-be-detected sample is prepared.

The first pool body 207 defines an optical detection window configured to perform optical detection, such as optical detection performed based on transmitted light or optical detection performed based on scattered light.

A shape of a cross section of the first pool body 207 is the same as or different from a shape of a cross section of the second pool body 208. Alternatively, a height of the first pool body 207 is the same as or different from a height of the second pool body 208. The differentiated configuration may be foolproof, allowing assembling and identification to be achieved easily.

The present embodiment further provides a reagent kit including the kit body 100 and the aforementioned detection cup assembly. The kit body 100 is arranged with a mounting portion. The detection cup assembly is arranged with a mating portion. The mounting portion is connected to the mating portion.

The mating portion may be a protruding portion or a recessed portion arranged on a side surface of the detection cup assembly. The protruding portion or the recessed portion is mated with the mounting portion. Alternatively, the mating portion may be a positioning protrusion arranged on the detection cup assembly. The positioning protrusion is mated with the mounting portion. For example, a flange may be configured to be snapped with the insertion hole in the kit body 100.

The detection cup assembly in the present embodiment has a novel structure. Based on the specific detected item, a corresponding required reagent may be prepared and arranged on the cup holder 2072. In this way, a detection efficiency may be improved greatly, and the detected item may be determined flexibly.

In a seventeenth embodiment, as shown in FIG. 1 to FIG. 6, the present embodiment provides a reagent kit including the kit body 100 and the assembling seat 160.

The kit body 100 includes the front pool 120, the front pool 120 is arranged with the front-pool electrode 121. The assembling seat 160 is connected to the kit body 100. The assembling seat 160 is arranged with the rear-pool electrode 165. The front-pool electrode 121 and the rear-pool electrode 165 are spaced apart from each other.

In the present embodiment, an axis of the front-pool electrode 121 and an axis of the rear-pool electrode 165 are substantially extending along a same straight line. It has been experimentally verified that, the detection accuracy is relatively high when the front-pool electrode 121 and the rear-pool electrode 165 are coaxially arranged. Each of the front-pool electrode 121 and the rear-pool electrode 165 is a column electrode. The manufacturing process and the assembling process of the column electrode are relatively simple.

The assembling seat 160 defines the axially-extending fluid guiding chamber 167. The front pool 120 and the axially-extending fluid guiding chamber 167 are communicated with each other through the micro-hole 172. The front-pool electrode 121 and the rear-pool electrode 165 are respectively disposed at two sides of the micro-porous slice 170.

The micro-porous slice 170 includes the slice body 171. The slice body 171 defines the micro-hole 172 that allows cells to pass through one by one. The micro-porous slice 170 is arranged on the assembling seat 160. Alternatively, the micro-porous slice 170 and one of the assembling seat 160 or the kit body 100 are configured as a one-piece and integral structure.

The axis of the front-pool electrode 121, the axis of the rear-pool electrode 165, and an axis of the micro-porous slice 170 are substantially extending along a same straight line.

An end of the front-pool electrode 121 may be protruding from, aligned with or recessed from an inner wall of the front pool 120. An end of the rear-pool electrode 165 may be protruding from, aligned with or recessed from a bottom wall of the axially-extending fluid guiding chamber 167. Two ends of the front-pool electrode 121 and the rear-pool electrode 165 are not particularly limited herein. The front-pool electrode 121 and the rear-pool electrode 165 contact the to-be-detected sample fluid while performing detection. The outer end of the front-pool electrode 121 and the outer end of the rear-pool electrode 165 are configured to an external power supply assembly.

The kit body 100 defines the mounting cavity 130. The assembling seat 160 includes the assembling cylinder 168, and the mounting cavity 130 is coaxially connected to the assembling cylinder 168.

The assembling cylinder 168 may be snap-fitted, threadly-fitted, interference-fitted, laser-welded, or adhesively-fitted with the mounting cavity 130.

The front-pool electrode 121 is arranged in the kit body 100 and may be protruding from, aligned with or recessed from an outer side of the kit body 100. The rear-pool electrode 165 is arranged on the assembling seat 160 and may be protruding from, aligned with or recessed from an outer side of the assembling seat 160.

The present embodiment further provides a sample detection device including the aforementioned reagent kit and the detection seat engaged with the reagent kit. The detection seat is configured to analyze and detect samples.

In an eighteenth embodiment, as shown in FIGS. 1 to 6, the present embodiment provides a reagent kit, the reagent kit includes the kit body 100 and a holding portion 150. The holding portion 150 is arranged on the kit body 100.

The holding portion 150 may be disposed near a center of a side of the kit body 100 or near a center of an upper surface of the kit body 100.

Two holding portions 150 may be arranged. The two holding portions 150 may be respectively disposed at two opposite sides of the kit body 100.

The holding portion 150 protrudes from the upper surface or the side of the kit body 100. An outer surface of the holding portion 150 is arranged with a snap protrusion 151 or defines a snap recess.

An anti-slip portion 152 is arranged on a top or an outer surface of the holding portion 150.

The holding portion 150 may be a resilient member. A side of the kit body 100 may be arranged with a recessed portion 154. The holding portion 150 is mated with the recessed portion 154.

The holding portion 150 and the recessed portion 154 may be configured as a one-piece and integral structure or may be detachably connected with each other.

The holding portion 150 is slidingly connected to the recessed portion 154 to allow the holding portion 150 to move relative to the kit body 100. The holding portion 150 may be extended and retracted relative to the kit body 100, such that the holding portion 150 may be stored easily, no space is occupied; and the holding portion 150 may be withdrawn when needed.

The present embodiment further provides a sample detection device including the reagent kit as previously described and the detection seat engaged with the reagent kit. The holding portion 150 is mated and connected with the detection seat.

In a nineteenth embodiment, as shown in FIG. 1 to FIG. 6, the present embodiment provides a reagent kit. The reagent kit includes the kit body 100 and a blocking portion 153.

The kit body 100 includes at least one pool position (such as the sample receiving pool 105). The pool position is configured to place a test tube (such as a sample tube 205).

The blocking portion 153 is disposed on a side of the pool position, configured to hook and fasten a cap (not shown in the drawings) of the test tube to prevent an opened cap from being reset to cover the opening of the test tube.

In an embodiment, the blocking portion 153 may be may be aligned with, protruding from, or recessed from the upper surface or the side surface of the kit body 100.

In an embodiment, the blocking portion 153 includes a connecting portion connected to the kit body 100 and a stopping portion bent relative to and extending from the connecting portion. An end of the stopping portion is arranged with a returning hook portion extending towards the kit body 100.

In another embodiment, the side surface of the kit body 100 is protruding outwardly to form the stopping portion 153. Alternatively, the stopping portion 153 may be a recess defined in the upper surface of the kit body 100. Alternatively, the upper surface of the kit body 100 defines a recess, and an inner wall of the recess serves as the stopping portion 153. The cap of the test tube, after being opened from the test tube, may be inserted into the recess. A size of the recess may be corresponding to a size of the cap of the test tube to achieve tight fitting.

The blocking portion 153 and the kit body 100 are may be configured as a one-piece and integral structure or may be detachably connected with each other. Specifically, the blocking portion 153 is snapped to, inserted into, threaded with the kit body 100, or is detachably connected to the kit body 100 through screws/pins.

In other embodiments, the blocking portion 153 is also slidingly connected to the kit body 100.

In the present embodiment, the pool position (such as the sample receiving pool 105) is located near an edge of the kit body 100. The blocking portion 153 is disposed at the edge of the kit body 100.

The present embodiment further provides a sample detection device including the aforementioned reagent kit and the detection seat engaged with the reagent kit. The detection seat is configured to analyze samples.

The above shows only embodiments of the present disclosure, and is not intended to limit the scope of the present disclosure. Any equivalent structure or equivalent process transformation performed based on the contents of the specification and the accompanying drawings of the present disclosure, being directly or indirectly applied in other related technical fields, shall be included in the scope of the present disclosure.

## Claims

1. A reagent kit, comprising: a kit body and an assembling seat, wherein the assembling seat comprises:
an assembling cylinder, defines an axially-extending fluid guiding chamber and a radially-extending fluid outlet communicated with the axially-extending fluid guiding chamber, wherein the radially-extending fluid outlet is configured to discharge a gas or a liquid in the axially-extending fluid guiding chamber; and
a rear-pool electrode, connected to the assembling cylinder and extending into the axially-extending fluid guiding chamber.

2. The reagent kit according to claim 1, wherein an outer surface of the assembling cylinder defines a recess communicated with the radially-extending fluid outlet, and the recess serves as a fluid guiding slot.

3. The reagent kit according to claim 1, wherein the assembling cylinder is arranged with a snap portion, and the snap portion is aligned with the radially-extending fluid outlet.

4. The reagent kit according to claim 1, wherein,
a first predetermined distance is defined between an inner end of the radially-extending fluid outlet and an inner end of the assembling cylinder;
a second predetermined distance is defined between an inner end of the rear-pool electrode and the inner end of the assembling cylinder; and
the first predetermined distance is less than or equal to the second predetermined distance.

5. The reagent kit according to claim 1, wherein a length of the radially-extending fluid outlet in an axial direction of the axially-extending fluid guiding chamber is greater than or equal to a length of the rear-pool electrode being received in the axially-extending fluid guiding chamber.

6. The reagent kit according to claim 1, wherein a micro-porous slice, which allows cells to pass through one by one, is arranged at an inner end of the assembling cylinder.

7. The reagent kit according to claim 6, wherein a third predetermined distance is defined between an inner end of the rear-pool electrode and the micro-porous slice, the third predetermined distance is 0.2-2 times of an axial length of the axially-extending fluid guiding chamber.

8. The reagent kit according to claim 6, wherein the inner end of the assembling cylinder is arranged with a sink, and the micro-porous slice is mated with the sink.

9. The reagent kit according to claim 3, wherein the assembling cylinder includes a rear-pool cylinder, an end plate, and an outer cylinder; the rear-pool cylinder, the end plate, and the outer cylinder are configured as a one-piece and integral structure; the rear pool defines the axially-extending fluid guiding chamber and the radially-extending fluid outlet; the end plate radially connects the rear-pool cylinder with the outer cylinder; the outer cylinder sleeves an outer periphery of the rear-pool cylinder and is spaced apart from the outer periphery of the rear-pool cylinder, the snap portion is disposed corresponding to a positioning bump.

10. A reagent kit, comprising:
a kit body, comprising a front pool and a pressure pool communicated with the front pool;
a assembling seat, comprising a assembling cylinder, wherein the assembling cylinder defines an axially-extending fluid guiding chamber and is connected to the kit body, and the front pool and the axially-extending fluid guiding chamber are communicated with each other and cooperatively serve as a fluid guiding channel.

11. The reagent kit according to claim 10, wherein,
the kit body defines a mounting cavity communicated with the front pool and the pressure pool; the assembling cylinder defines the axially-extending fluid guiding chamber; the front pool, the mounting cavity, the axially-extending fluid guiding chamber, the radially-extending fluid outlet, and the pressure pool cooperatively serve as the fluid guiding channel; the assembling seat further comprises a rear-pool electrode, the rear-pool electrode is connected to the assembling cylinder and extends into the axially-extending fluid guiding chamber.

12. The reagent kit according to claim 10, wherein an outer surface of the assembling cylinder defines a recess communicated with the radially-extending fluid outlet, and the recess serves as a fluid guiding slot.

13. The reagent kit according to claim 10, wherein the assembling cylinder is arranged with a snap portion, and the snap portion is aligned with the radially-extending fluid outlet.

14. The reagent kit according to claim 11, wherein,
a first predetermined distance is defined between an inner end of the radially-extending fluid outlet and an inner end of the assembling cylinder;
a second predetermined distance is defined between an inner end of the rear-pool electrode and the inner end of the assembling cylinder; and
the first predetermined distance is less than or equal to the second predetermined distance.

15. The reagent kit according to claim 11, wherein a length of the radially-extending fluid outlet in an axial direction of the axially-extending fluid guiding chamber is greater than or equal to a length of the rear-pool electrode being received in the axially-extending fluid guiding chamber.

16. The reagent kit according to claim 10, wherein a micro-porous slice, which allows cells to pass through one by one, is arranged at an inner end of the assembling cylinder; the micro-porous slice comprises a slice body, the slice body defines a micro-hole that allows cells to pass through one by one; and a reinforcing portion is further arranged on the slice body.

17. The reagent kit according to claim 16, wherein the slice body is a plastic slice body or a ceramic slice body.

18. The reagent kit according to claim 16, wherein the reinforcing portion is a convex ring; the convex ring is configured as a continuous one-piece convex ring or is formed by a plurality of protrusions cooperatively; and an outer edge of the convex ring coincides with or dis-coincides with an outer edge of the slice body.

19. The reagent kit according to claim 16, wherein an inner end of the assembling cylinder is arranged with a sink, the micro-porous slice is mated with the sink.

20. The reagent kit according to claim 10, wherein the assembling cylinder includes a rear-pool cylinder, an end plate, and an outer cylinder; the rear-pool cylinder, the end plate, and the outer cylinder are configured as a one-piece and integral structure; the rear-pool cylinder defines the axially-extending fluid guiding chamber and the radially-extending fluid outlet; the end plate radially connects the rear-pool cylinder with the outer cylinder; the outer cylinder sleeves an outer periphery of the rear-pool cylinder and is spaced apart from the outer periphery of the rear-pool cylinder.

21. A sample detection device, comprising the reagent kit according to claim 1 and a detection seat engaged with the reagent kit, wherein the sample detection device is configured to analyze and detect samples.
